# EUROPÄISCHE PATENTSCHRIFT

(11) **EP 0 765 419 B1**
(45) Veröffentlichungstag und Bekanntmachung des Hinweises auf die Patenterteilung: **07.06.2006**
(21) Anmeldenummer: 95922508.7
(22) Anmeldetag: 06.06.1995
(51) Int. Cl.: D21H 19/00, B29C 33/60, C10M 173/00, C10M 105/24, C10M 129/40, C10N 40/36

(54) **VERFAHREN ZUR HERSTELLUNG VON WÄSSRIGEN METALLSEIFENDISPERSIONEN**
PROCESS FOR PREPARING AQUEOUS METALLIC SOAP DISPERSIONS
PROCEDE DE PREPARATION DE DISPERSIONS AQUEUSES DE SAVONS METALLIQUES

(30) Priorität: 13.06.1994 DE 4420517
(43) Veröffentlichungstag der Anmeldung: 02.04.1997
(73) Patentinhaber: Cognis Deutschland GmbH & Co. KG, 40789 Monheim (DE)
(72) Erfinder: ROSSELO BLASI, Antoni, E-08690 Barcelona (ES); BIGORRA LLOSAS, Joaquim, E-08203 Sabadell (ES); PI SUBIRANA, Rafael, E-08400 Granollers (ES)
(86) Internationale Anmeldenummer: PCT/EP1995/002147
(87) Internationale Veröffentlichungsnummer: WO 1995/034717

(56) Entgegenhaltungen:
- WO-A-91/01962
- WO-A-91/12371
- DD-A- 155 770
- DE-A- 3 403 621
- DE-C- 829 296
- US-A- 4 659 489
- DATABASE WPI Section Ch, Week 8606 Derwent Publications Ltd., London, GB; Class A97, AN 86-038615 & JP-A-60 258 139 ( NIPPON OILS & FATS KK) , 20.Dezember 1985
- DATABASE WPI Section Ch, Week 8418 Derwent Publications Ltd., London, GB; Class A97, AN 84-110788 & JP-A-59 051 236 ( SAN NOPCO KK) , 24.März 1984

## Beschreibung

### Gebiet der Erfindung

Die Erfindung betrifft ein verbessertes Verfahren zur Herstellung von wäßrigen Metallseifendispersionen, insbesondere Calciumstearatdispersionen, bei dem man in eine wäßrige Metallsalzlösung ausgewählte Fettsäuregemische einrührt, ausgewählte Metallseifendispersionen sowie die Verwendung der wäßrigen Metallseifendispersionen.

### Stand der Technik

Metallseifen, wie beispielsweise die Barium-, Calcium-, Cadmium- und Zinksalze der Palmitin- und/oder Stearinsäure werden häufig als Gleit- und Trennmitteladditive eingesetzt [US 4659489, Henkel Corp.). In der Regel werden die Metallseifen dabei in Form hochkonzentrierter wäßriger Slurries verwendet, die sich im Gegensatz zu Pulvern leichter bevorraten und dosieren lassen.

Das rheologische Verhalten bzw. die Viskosität derartiger Feststoffdispersionen ist im Gegensatz zu einfachen newtonschen Flüssigkeiten in vielen Fällen davon abhängig, welcher Scherbelastung die Probe ausgesetzt wird. Beim Handling der Dispersionen durch den Anwender treten beim Auslaufen, Rühren, Pumpen und Filtrieren die unterschiedlichsten Schergefälle auf, die sich über einen Schergeschwindigkeitsbereich von 10⁻¹ bis 10⁴ s⁻¹ erstrecken können. Häufig findet man bei Dispersionen ein Fließverhalten, bei dem mit steigender Schergeschwindigkeit eine Abnahme der Viskosität resultiert; zusätzlich können Fließgrenzen existieren, so daß erst ein bestimmtes Maß an Schubspannung überschritten werden muß, um die Dispersion zum Fließen zu veranlassen. Neben diesem strukturviskosen, plastischen bzw. pseudoplastischen Verhalten können sich wäßrige Metallsalzslurries auch dilatant verhalten, d.h., mit zunehmender Schergeschwindigkeit insbesondere hochkonzentrierter Dispersionen wird eine Zunahme der Viskosität beobachtet. In einigen Fällen kann schließlich noch ein zeitabhängiges Fließverhalten festgestellt werden, wie das Auftreten einer Thixotropie. Eine Übersicht zu diesen Phaenomenen von B.Burg und R.Jeschke findet sich in Fat Sci. Technol., 94, 249 (1992).

In diesem Zusammenhang besteht ein Bedürfnis nach wäßrigen Metallseifendispersionen, die sich trotz eines hohen Feststoffgehaltes durch niedrige Viskosität und Fließgrenze sowie verbesserte Filtrierbarkeit auszeichnen und auch bei Scherung nicht verdicken.

Ein weiteres bisher nicht zur Zufriedenheit gelöstes Problem besteht darin, daß beim Auftragen der Dispersionen auf die

DD-A-155 770 offenbart, daß freie Iso-Stearinsäure in Mischung mit weiteren Stoffen die Viskosität von Metallseifen dispersionen herabsetzen kann. Über mischungen von Linearen und verzweigten Fettsäuren und deren Wirkung auf Metallseifen dispersionen wird nidits offenbart.

DE-A-34 03621 betrifft ein Verfahren zur Herstellung von Metallseifen. Dabei wird eine kleine Menge von polymeren Fettsäuren, einer alphatischen Dicarbonsäure, einer aus der Ozonisierung von Oleinsäure erhaltenen Säure eingesetzt.

DE-C-829 296 beschreibt ein Verfahren zur Abtrennung geradkettiger Fettsäuren aus isocarbonsäurehaltigen Fettsäure gemischen. Gemäß den beiden Ausführungsbeispielen dieser Patentschrift werden Dicarbonsäure-haltige Fettsäuregemische eingesetzt.

Papierbahn starke Turbulenzen auftreten, die zur Streifenbildung auf dem fertigen Papier führen. Folglich besteht ein weiteres Marktbedürfnis nach Produkten, mit denen dieser unerwünschte Effekt zuverlässig verhindert werden kann.

Die Aufgabe der Erfindung hat somit darin bestanden, Metallseifendispersionen dieses komplexen Anforderungsprofils zur Verfügung zu stellen.

### Beschreibung der Erfindung

Gegenstand der Erfindung ist ein Verfahren zur Herstellung von wäßrigen Metallseifendispersionen, das sich dadurch auszeichnet, daß man eine wäßrige Lösung eines Metalloxids bzw. -hydroxid vorlegt und eine Mischung bestehend aus
a) einer linearen Fettsäure und
b) einer verzweigten Fettsäure
und gegebenenfalls gegen Ende des Fettsäurezugabe Additive einrührt.

Überraschenderweise wurde gefunden, daß bereits ein geringer Zusatz der verzweigten Fettsäuren zu Dispersionen führt, die gegenüber den Produkten des Stands der Technik eine signifikant niedrigere Viskosität aufweisen. Die Erfindung schließt die Erkenntnis ein, daß die nach dem vorgeschlagenen Verfahren erhältlichen Dispersionen zudem niedrigere Fließgrenzen aufweisen, auch bei starker Scherung nicht verdicken und eine stark verbesserte Filtrierbarkeit zeigen.

### Metalloxide, -hydroxide und -seifen

Unter den erfindungsgemäßen Metallseifen sind Dispersionen von Calcium-, Barium-, Cadmium- und/ oder Zinkseifen zu verstehen. Im Sinne des erfindungsgemäßen Verfahrens werden wäßrige Lösungen von Metalloxiden bzw. -hydroxiden wie beispielsweise von Calciumoxid, Calciumhydroxid, Bariumoxid, Bariumhydroxid, Cadmiumoxid und/oder Zinkoxid vorgelegt, die typischerweise einen Feststoffgehalt im Bereich von 5 bis 60 und insbesondere 40 bis 55 Gew.-% aufweisen. Vorzugsweise werden 40 bis 60 Gew.-%ige wäßrige Lösungen von Calciumoxid bzw. -hydroxid eingesetzt.

### Fettsäuren

Als Komponente a) kommen Fettsäuren der Formel (I) in Frage,

R¹CO-OH (I)

in der R¹CO für einen linearen, gesättigten und/oder ungesättigten Acylrest mit 12 bis 22 und insbesondere 16 bis 18 Kohlenstoffatomen steht. Typische Beispiele sind Capronsäure, Caprylsäure, Caprinsäure, Laurinsäure, Myristinsäure, Palmitinsäure, Palmoleinsäure, Stearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und ölen oder bei der Reduktion von Aldehyden aus der Roelen'schen Oxosynthese anfallen. Bevorzugt sind technische Fettsäuren mit 16 bis 18 Kohlenstoffatomen wie beispielsweise Talgfettsäure und insbesondere technische bzw. reine Stearinsäure.

Als Komponente b) kommen schließlich Fettsäuren der Formel (II) in Betracht,

R²CO-OH (II)

in der R²CO für einen verzweigten, gesättigten und/oder ungesättigten Acylrest mit 6 bis 22 Kohlenstoffatomen steht. Typische Beispiele sind 2-Ethylhexylsäure, Isotridecylsäure, Isopalmitinsäure, Isostearinsäure und Isoölsäure sowie deren technische Gemische mit den entsprechenden linearen Homologen. Vorzugsweise wird technische Isostearinsäure mit einem Gehalt an verzweigten Fettsäuren im Bereich von 10 bis 95 und vorzugsweise 25 bis 75 Gew.-% eingesetzt.

Die linearen und verzweigten Fettsäuren, die die Komponenten a) und b) bilden, können im Gewichtsverhältnis 50 : 50 bis 99,5 : 0,5 und insbesondere 90 : 10 bis 99 : 1 eingesetzt werden. Die Menge an einzusetzendem Fettsäuregemisch ergibt sich aus der vorgelegten Menge der Base und sollte üblicherweise zur Neutralisation ausreichen; ein ca. 10 Gew.-% Über- bzw. Unterschuß an Fettsäure ist jedoch tolerabel.

In Summe bevorzugt ist die Herstellung einer wäßrigen Calciumstearatdispersion mit einem Feststoffgehalt im Bereich von 45 bis 55 Gew-%, bei dem man einer entsprechenden Lösung von Calciumhydroxid in Wasser eine Mischung aus Stearinsäure und technischer Isostearinsäure (Gewichtsverhältnis 95 : 5 bis 99 : 1) zusetzt. Die Partikelgrößenverteilung der Metallseifendispersionen ist nicht von ausschlaggebender Bedeutung. Gewisse Vorteile werden jedoch für solche Produkte gesehen, bei denen mehr als 50 Vol-% der Teilchen einen Durchmesser oberhalb von 3,5 µm aufweisen.

### Additive

Zur zusätzlichen Verbesserung des rheologischen Verhaltens hat es sich als vorteilhaft erwiesem, gegen Ende der Fettsäurezugabe bestimmte Hilfsstoffe zuzudosieren. Für diesen Zweck haben sich in der Vergangenheit bereits Nonylphenolethoxylate als nützlich erwiesen. Aus anwendungstechnischen Gründen, aber auch aus ökologischer Sicht, kommen in einer bevorzugten Ausführungsform der Erfindung als Rheologieadditive Mischungen, enthaltend
c) Anlagerungsprodukte von 1 bis 10 Mol Alkylenoxid an lineare und/oder verzweigte Alkohole mit 6 bis 22 Kohlenstoffatomen, Octylphenol oder Nonylphenol und
d) Anlagerungsprodukte von 1 bis 10 Mol Alkylenoxid an gesättigte und/oder ungesättigte Fettsäuren mit 12 bis 18 Kohlenstoffatomen
in Betracht. Typische Beispiele sind Mischungen von Anlagerungsprodukten von 1 bis 10 Mol Ethylenoxid an Nonylphenol oder insbesondere Octanol bzw. Kokosfettalkohol einerseits und ethoxylierten Fettsäuren wie beispielsweise Ölsäure-5 EO-Addukt andererseits. Das Gewichtsverhältnis der Komponenten c) und d) kann dabei im Bereich von 1 : 2 bis 2 : 1 liegen. Vorzugsweise werden die Entschäumer in Konzentrationen im Bereich von 1 bis 6, vorzugsweise 2 bis 5,5 Gew.-% - bezogen auf die Dispersion - eingesetzt.

Besonders bevorzugt ist ferner auch der Zusatz von Mischungen auf Basis von unterschiedlich hoch ethoxylierten verzweigten Alkoholen wie beispielsweise der Mischung eines Isododecylalkohol-6 EO- und eines Isododecylalkohol-12 EO-Adduktes im Gewichtsverhältnis von etwa 2 : 1.

In einer weiteren bevorzugten Ausführungsform der Erfindung können den Metallseifendispersionen Umesterungsprodukte von Triglyceriden mit Polyethylenglycolen als Additive zugesetzt werden, die das rheologische Verhalten auch von Dispersionen auf Basis reiner Stearinsäure weiter verbessern. Beispiele für geeignete Additive sind Umesterungsprodukte von 1 Mol Kokosöl, Palmöl, Palmkernöl, Olivenöl, Sonnenblumenöl oder Rindertalg mit 1 bis 3 Mol eines Polyethylenglycols mit einem durchschnittlichen Molekulargewicht von 100 bis 1000. Die Additive können in Mengen von 1 bis 5 und vorzugsweise 2 bis 3 Gew.-% - bezogen auf die Dispersionen - eingesetzt werden.

### Herstellung der Dispersionen

In einer bevorugten Ausführungsform der Erfindung wird der Calciumhydroxidlösung in einem Rührgefäß vorgelegt und die Fettsäuremischung unmittelbar auf einen sich in der Lösung befindlichen, rasch rötierenden Drehteller aufgegeben. Die dabei abgeschleuderten Fettsäuretröpfchen werden besonders fein verteilt und führen zur Bildung von Calciumsalzpartikeln einer vorteilhaften Partikelgröße.

### Gewerbliche Anwendbarkeit

Die nach dem erfindungsgemäßen Verfahren erhältlichen Metallseifendispersionen, insbesondere Calciumstearatdispersionen, sind niedrigviskos, besitzen niedrige Fließgrenzen, verdicken auch bei Scherung nicht und lassen sich leicht filtrieren.

Die folgenden Beispiele sollen den Gegenstand der Erfindung näher erläutern, ohne ihn darauf einzuschränken.

### Beispiele

### I. Eingesetzte Fettsäuren

Die Zusammensetzung der eingesetzten Fettsäuren ist in Tabelle 1 zusammengefaßt. Alle Prozentangaben verstehen sich als Gew.-%.

**Tabelle 1: Eingesetzte Fettsäuren**

| **Fettsäure** | **A** | **B1** | **B2** | **B3** |
|---|---|---|---|---|
| | **%** | **%** | **%** | **%** |
| Myristinsäure | - | 2 | - | 1 |
| Palmitinsäure | - | 26 | 8 | 33 |
| Stearinsäure | 100 | 10 | 6 | 51 |
| Isostearinsäure | - | 30 | 3 | 10 |
| Ölsäure | - | 28 | 46 | 5 |
| Isoölsäure | - | 3 | 37 | - |
| Arachinsäure | - | 1 | - | - |

| | | | | |
|---|---|---|---|---|
| Legende: B1 = EMERSOL^{(R)} 935, Henkel Corp., Cincinnati/USA | | | | |
| B2 = CENTURY^{(R)} MOS, Union Camp Chem./GB | | | | |
| B3) ALIPHAT^{(R)} 47, Unichema, Emmerich/FRG . | | | | |

### II. Rheologisches Verhalten

In eine wäßrige Lösung von Calciumhydroxid wurde eie Mischung aus Stearinsäure und technischer Isostearinsäure sowie gegebenenfalls einem Additiv eingerührt.

Der Feststoffgehalt der Lösung betrug nach vollständiger Zugabe der Fettsäure 50 Gew.-%. Gegen Ende der Herstellung wurde der Dispersion eine 1 : 1 Mischung aus einem Anlagerungsprodukt von durchschnittlich 10 Mol Ethylenoxid an Nonylphenol und einem Ölsäure-5,5 EO-Addukt in einer Gesamtmenge von etwa 5 Gew.-% - bezogen auf die Dispersion - als Entschäumer zugesetzt.

Die Viskosität wurde in einem Hrookfield-Viskosimeter (Spindel 2, 50 Upm, 25°C) und das thixotrope Verhalten in einem konventionellen Rheomaten (25°C) bestimmt. Die Ergebnisse sind in Tabelle 2 zusammengefaßt. Die Prozentangaben verstehen sich wieder als Gew.%.

**Tabelle 2: Viskosität und Thixotropie von Calciumstearat-Dispersionen**

| **Bsp.** | **c(A) %** | **B** | **c(B)** | **c(C) %** | **Viskosität % mPa*s** | **ThixotropiePa*s** |
|---|---|---|---|---|---|---|
| 1 | 96 | B1 | 4 | - | 104 | + 4337 |
| 2 | 98 | B1 | 2 | - | 66 | + 2221 |
| 3 | 98 | B2 | 2 | - | 78 | + 2005 |
| 4 | 98 | B3 | 2 | - | 99 | + 1891 |
| 5 | 94 | B1 | 2 | 2 | 113 | + 4346 |
| 6 | 96 | B1 | 2 | 2 | 107 | + 3328 |
| V1 | 100 | - | - | - | 138 | - 42305 |
| V2 | 98 | - | - | 2 | 205 | + 5147 |

| | | | | | | |
|---|---|---|---|---|---|---|
| Legende: c(A) = Konzentration Stearinsäure | | | | | | |
| c(B) = Konzentration Isostearinsäure | | | | | | |
| c(C) = Konzentration Additiv : Umesterungsprodukt von Olivenöl mit Polyethylenglycol (M = 1000) | | | | | | |

### III. Filtrierbarkeit

Zur Beurteilung der Filtrierbarkeit wurden die Dispersionen gemäß Beispiel 1 und Vergleichsbeispiel V1 durch einen Seitz-Filter filtriert und der Durchfluß als Funktion des Drucks bestimmt. Die Ergebnisse sind in Tabelle 3 zusammengefaßt.

**Tabelle 3 Filtrierbarkeit**

| **Bsp.** | **c(A)** | **B** | **c(B)** | **Druck** | **Durchfluß** |
|---|---|---|---|---|---|
| | | | | **bar** | **l/min** |
| 1 | 96 | B1 | 4 | 0,2 | 8,0 |
| V1 | 100 | - | - | 0,2 | 0,2 |
| | | | | 2,0 | 2,0 |
| | | | | 4,0 | 3,2 |
| | | | | 6,0 | 5,2 |
| | | | | 8,0 | 5,8 |

## Patentansprüche

1. Verfahren zur Herstellung von wässrigen Metallseifendispersionen, **dadurch gekennzeichnet, dass** man eine wässrige Lösung eines Metalloxids bzw. -hydroxids vorlegt und eine Mischung bestehend aus
a) einer linearen Fettsäure und
b) einer verzweigten Fettsäure
und gegebenenfalls gegen Ende der Fettsäurezugabe Additive
einrührt.

2. Verfahren nach Anspruch 1, **dadurch gekennzeichnet, dass** man Dispersionen von Calcium-, Barium-, Cadmium- und/ oder Zinkseifen herstellt.

3. Verfahren nach den Ansprüchen 1 und 2, **dadurch gekennzeichnet, dass** man als Metalloxide bzw. -hydroxide Calciumoxid, Calciumhydroxid, Bariumoxid, Bariumhydroxid, Cadmiumoxid und/oder Zinkoxid einsetzt.

4. Verfahren nach den Ansprüchen 1 bis 3, **dadurch gekennzeichnet, dass** die wässrigen Lösungen der Metalloxide bzw. -hydroxide einen Feststoffgehalt im Bereich von 5 bis 60 Gew.-% aufweisen.

5. Verfahren nach den Ansprüchen 1 bis 4, **dadurch gekennzeichnet, dass** man als Komponente a) Fettsäuren der Formel (I) einsetzt,
**R**^{**1**}**CO-OH** **(I)**
in der R¹CO für einen linearen, gesättigten und/oder ungesättigten Acylrest mit 12 bis 22 Kohlenstoffatomen steht.

6. Verfahren nach den Ansprüchen 1 bis 5, **dadurch gekennzeichnet, dass** man als Komponente b) Fettsäuren der Formel (II) einsetzt,
**R**^{**2**}**CO-OH** **(II)**
in der R²CO für einen verzweigten, gesättigten und/oder ungesättigten Acylrest mit 6 bis 22 Kohlenstoffatomen steht.

7. Verfahren nach den Ansprüchen 1 bis 6, **dadurch gekennzeichnet, dass** man die Komponenten a) und b) im Gewichtsverhältnis 50 : 50 bis 99,5 : 0,5 einsetzt.

8. Verfahren nach den Ansprüchen 1 bis 7, **dadurch gekennzeichnet, dass** man als Additive Mischungen, enthaltend
c) Anlagerungsprodukte von 1 bis 10 Mol Alkylenoxid an lineare und/oder verzweigte Alkohole mit 6 bis 22 Kohlenstoffatomen, Octylphenol oder Nonylphenol und
d) Anlagerungsprodukte von 1 bis 10 Mol Alkylenoxid an gesättigte und/oder ungesättigte Fettsäuren mit 12 bis 18 Kohlenstoffatomen
einsetzt.

9. Verfahren nach den Ansprüchen 1 bis 7, **dadurch gekennzeichnet**, das man als Additive Mischungen unterschiedlich hoch ethoxylierter verzweigter Alkohole einsetzt.

10. Verfahren nach den Ansprüchen 1 bis 7, **dadurch gekennzeichnet, das** man als Additive Umesterungsprodukte von Triglyceriden mit Polyethylenglycol einsetzt.

## Claims

1. A process for the production of aqueous metal soap dispersions, **characterized in that** an aqueous solution of a metal oxide or hydroxide is initially introduced and a mixture of
a) a linear fatty acid and
b) a branched fatty acid
and, optionally towards the end of the addition of the fatty acids, additives are stirred in.

2. A process as claimed in claim 1, **characterized in that** dispersions of calcium, barium, cadmium and/or zinc soaps are prepared.

3. A process as claimed in claims 1 and 2, **characterized in that** calcium oxide, calcium hydroxide, barium oxide, barium hydroxide, cadmium oxide and/or zinc oxide are used as the metal oxides or hydroxides.

4. A process as claimed in claims 1 to 3, **characterized in that** the aqueous solutions of the metal oxides or hydroxides have a solids content of 5 to 60% by weight.

5. A process as claimed in claims 1 to 4, **characterized in that** fatty acids corresponding to formula (I):
**R**^{**1**}**CO-OH** **(I)**
in which R¹CO is a linear, saturated and/or unsaturated acyl group containing 12 to 22 carbon atoms,
are used as component a).

6. A process as claimed in claims 1 to 5, **characterized in that** fatty acids corresponding to formula (II):
**R**^{**2**}**CO-OH** **(II)**
in which R²CO is a branched, saturated and/or unsaturated acyl group containing 6 to 22 carbon atoms,
are used as component b).

7. A process as claimed in claims 1 to 6, **characterized in that** components a) and b) are used in a ratio by weight of 50:50 to 99.5:0.5.

8. A process as claimed in claims 1 to 7, **characterized in that** mixtures containing
c) products of the addition of 1 to 10 mol alkylene oxide onto linear and/or branched alcohols containing 6 to 22 carbon atoms, octylphenol or nonylphenol and
d) products of the addition of 1 to 10 mol alkylene oxide onto saturated and/or unsaturated fatty acids containing 12 to 18 carbon atoms
are used as the additives.

9. A process as claimed in claims 1 to 8, **characterized in that** mixtures of branched alcohols with different degrees of ethoxylation are used as the additives.

10. A process as claimed in claims 1 to 9, **characterized in that** transesterification products of triglycerides with polyethylene glycol are used as the additives.

## Revendications

1. Procédé de préparation de dispersions aqueuses de savons métalliques,
**caractérisé en ce qu'**
on met en oeuvre une solution aqueuse d'un oxyde ou d'un hydroxyde métallique et on y ajoute, sous agitation, un mélange constitué de
a) un acide gras linéaire et
b) un acide gras ramifié
et, le cas échéant des additifs vers la fin de l'addition des acides gras.

2. Procédé selon la revendication 1,
**caractérisé en ce qu'**
on prépare des dispersions de savons de calcium, de baryum, de cadmium et/ ou de zinc.

3. Procédé selon les revendications 1 et 2,
**caractérisé en ce qu'**
on utilise, comme oxydes ou hydroxydes métalliques, de l'oxyde de calcium, de l'hydroxyde de calcium, de l'oxyde de baryum, de l'hydroxyde de baryum, de l'oxyde de cadmium et/ou de l'oxyde de zinc.

4. Procédé selon les revendications 1 à 3,
**caractérisé en ce que**
les solutions aqueuses des oxydes ou des hydroxydes métalliques présentent une teneur en substances solides de l'ordre de 5 à 6 % en poids.

5. Procédé selon les revendications 1 à 4,
**caractérisé en ce qu'**
on utilise, comme composant a), des acides gras de formule (I),
R¹CO-OH (I)
dans laquelle R¹CO représente un radical acyle linéaire, saturé et/ou insaturé comportant 12 à 22 atomes de carbone.

6. Procédé selon les revendications 1 à 5,
**caractérisé en ce qu'**
on utilise, comme composant b) des acides gras de formule (II)
R²CO-OH (II)
dans laquelle R²CO représente un radical acyle ramifié, saturé et/ou insaturé comportant 6 à 22 atomes de carbone.

7. Procédé selon les revendications 1 à 6,
**caractérisé en ce qu'**
on utilise les composants a) et b) dans un rapport pondéral de 50:50 à 99,5:0,5.

8. Procédé selon les revendications 1 à 7,
**caractérisé en ce qu'**
on utilise, comme additifs, des mélanges contenant
c) des produits d'addition de 1 à 10 moles d'oxyde d'alkylène sur des alcools linéaires et/ou ramifiés comportant 6 à 22 atomes de carbone, de l'octylphénol ou du nonylphénol et
d) des produits d'addition de 1 à 10 moles d'oxyde d'alkylène sur des acides gras saturés et/ou insaturés comportant 12 à 18 atomes de carbone.

9. Procédé selon les revendications 1 à 7,
**caractérisé en ce qu'**
on utilise, comme additifs, des mélanges d'alcools ramifiés différemment éthoxylés.

10. Procédé selon les revendications 1 à 7,
**caractérisé en ce qu'**
on utilise, comme additifs, des produits de transestérification de triglycérides avec du polyéthylène glycol.
